# EUROPEAN PATENT APPLICATION

(11) **EP 1 808 495 A1**
(43) Date of publication of application: **18.07.2007**
(21) Application number: 06100270.5
(22) Date of filing: 12.01.2006
(51) Int. Cl.: C12Q 1/68, G01N 33/68, C12N 15/10

(54) **Method and kit for detecting interactions between transcriptionally active proteins**

(71) Applicant: Dualsystems Biotech AG, 8057 Zürich (CH)
(72) Inventor: Auerbach, Daniel, 8005 Zürich (CH)
(74) Representative: Becker, Konrad

(57) **Abstract**

The present invention is concerned with a method and a kit for detecting an interaction between a first test protein or fragment thereof which is artificially bound to a cell membrane and a second test protein or fragment thereof which is either membrane bound or soluble with an in vivo genetic system based in yeast, bacterial or mammalian cells. The system makes use of the reconstitution of the split ubiquitin protein.

## Description

### Field of the invention

The present invention relates to a method for detecting protein-protein interactions with an in vivo genetic system. The method is based on the use of split-ubiquitin chimeric genes encoding hybrid proteins.

### Background of the invention

An important area in biology is the analysis of interactions between proteins. Proteins are complex macromolecules made up of covalently linked chains of amino acids. Each protein assumes a unique three-dimensional shape principally determined by its sequence of amino acids. Many proteins consist of smaller units called domains, which are continuous stretches of amino acids able to fold independently from the rest of the protein (e. g. alpha-helices, beta-sheets).

Interactions between proteins mediate most processes in a living cell. They are involved, for example, in the assembly of enzyme subunits, antigen-antibody reaction, and in forming the supramolecular structures of ribosomes, filaments and viruses.

Protein-protein interactions have been generally studied in the past ten years by using biochemical techniques such as crosslinking, co-immunoprecipitation and co-fractionation by chromatography. Biochemical methods have the disadvantage that interacting proteins are generally recognized as bands of a particular mobility on a polyacrylamide gel. To progress from these bands to cloned genes is often a very tedious process.

In recent years, the discovery and characterization of protein interactions on a large scale has helped to elucidate many novel biological pathways. Due to their relative speed and ease of use, genetic screening systems such as the yeast two-hybrid system have been especially successful in finding novel protein interactions.

The yeast two-hybrid system has been developed in 1989 by Stan Fields and Ok-Kyu Song (Fields, S. and Song, O.K., 1989. Nature 340:245-248). This system is based on reconstitution of a transcriptional activator (a "bait" protein is fused to the DNA-binding domain and a "prey" protein to the transcription activation domain) and transcriptional activation of reporter genes. The yeast two-hybrid system is a powerful method in the in vivo analysis of the protein-protein interaction.

However, due to its nature, the yeast two-hybrid system is biased against certain classes of proteins, such as proteins containing highly acidic regions or transcription factors (Fashena, S.J., I. Serebriiskii, and E.A. Golemis. 2000. Gene 250:1-14). When fused to a DNA-binding domain, these proteins often autonomously activate transcription and therefore cannot be used in a yeast two-hybrid assay. One such example is the tumor suppressor protein p53, which requires removal of its N-terminal transactivation domain for use with the yeast two-hybrid system (Schmieg, F.I. and D.T. Simmons. 1984. J. Virol. 52:350-355.)

The split-ubiquitin system represents an alternative assay for the in vivo analysis of protein interactions. It was developed in 1994 by Niels Johnsson and Alexander Varshavsky (Johnsson, N. and Varshavsky, A. 1994. Proc. Natl. Acad. Sci. USA 91:10340-10344) for the detection of interactions between soluble proteins. Ubiquitin (Ub) is a 76 amino acid residue, single domain protein that is present in cells either free or covalently linked to other proteins. Ubiquitin plays a role in a number of processes primarily through routes that involve protein degradation. In eukaryotes, newly formed Ub fusions are rapidly cleaved by ubiquitin specific proteases (UBPs) after the last residue of Ub at the Ub-polypeptide junction. The cleavage of a UB fusion by UBPs requires the folded conformation of Ub. When a C-terminal fragment of ubiquitin (CbM) is expressed as a fusion to a reporter protein, the fusion is cleaved only if an N-terminal fragment of Ubiquitin (NbM) is also expressed in the same cell. This reconstitution of native ubiquitin from its fragments, detectable by the in vivo cleavage assay, is not observed with a mutationally altered NbM. However, if CbM and the altered NbM are each linked to polypeptides that interact in vivo, the cleavage of the fusion containing CbM is restored, yielding a generally applicable assay for detecting the protein-protein interactions. This method can be used for finding interacting partners to transcriptionally active proteins, however the method itself has several disadvantages. For example, this system uses resistance to 5-fluoro-orotic acid (5-FOA) as the selection mechanism. 5-FOA selection is difficult to apply in cDNA library screens as it produces a high number of false positives due to non-specific growth in the presence of 5-FOA. Furthermore, a replica plating step must be used for selection, which makes the method very laborious.

The system was subsequently modified to use a different sort of reporter and it was shown to work with membrane proteins (Stagljar et al. 1998. Proc. Natl. Acad. Sci. USA 95:5187-5192). Three yeast membrane proteins of the endoplasmic reticulum were used as a model system to show that the modified split-ubiquitin system works as a detection system for interactions involving membrane-associated proteins. In contrast to the conventional two-hybrid system, which requires nuclear localization, the interactions are detected in their natural environment of the protein of interest. Upon interaction of the proteins, the transcription factor is cleaved off and allowed to move into the nucleus where it activates a reporter gene causing a positive signal. According to this prior art, the constructs used for establishing the split-ubiquitin system were designed for integration into the host genome.

The system was further adapted and made suitable for high-scale screenings (WO03/083136). This document suggests that in addition to membrane protein interactions, also protein-protein interactions of transcriptionally active proteins could be detected by the disclosed method, if such proteins were to be artificially attached to the membrane by e.g. a fatty acid modification. Various signals for fatty acid modifications are known (some of them are listed in Solski et al. 1995. Methods in Enzymol. 250:435-54 or in Wedegaertner et al. 1995. J. Biol. Chem. 270:503-506). However, since fatty acid modification is carried out post-translationally, an amount of unmodified bait protein might manage to escape to the nucleus and cause the activation of the reporter gene by itself. Also the fact that fatty acid modifications are reversible means that there will be an equilibrium of membrane-bound and non-bound bait proteins in the cell, making a screening procedure very difficult.

To overcome these problems a new genetic method was developed which is disclosed here.

### Summary of the invention

The method of the invention is useful for the detection of in vivo interactions of transcriptionally active proteins and proteins that cannot be analysed by other methods. It uses targeting of such proteins to the membrane by fusing the proteins to integral membrane proteins or membrane protein domains. Fusion to such membrane proteins leads to artificial attachment of a protein to the membrane. This method can be used with full-length proteins containing acidic regions or transactivation domains. Further advantage of the method is the fact that it is suitable for both cytosolic and nuclear proteins. Even if the nuclear protein contains a strong nuclear localization signal, it will not be able to translocate to the nucleus as it is artificially attached to the membrane. As opposed to other genetic interaction assays, the "bait" remains in the cytosol since it is fused to the membrane anchor and never moves to the nucleus, thereby avoiding transcriptional activation of the reporter genes in the absence of a bait-prey interaction. Therefore, the method is very flexible and can be used with essentially any soluble protein, protein subfragment or domain.

It is therefore an object of the present invention to provide a method for detecting in vivo protein interactions of a wide range of transcriptionally active proteins and other proteins which are not suitable for being screened by standard methods, such as e.g. by a classical yeast two-hybrid method. The method of the invention is characterized in that such protein is artificially attached to the membrane. This can be achieved by fusing this protein to a membrane protein or a domain which has its C-terminus at the cytoplasmic side of the membrane. This membrane protein or domain can be any eukaryotic or prokaryotic integral membrane protein or membrane-associated protein or a domain or a fragment of a membrane protein having its C-terminal end facing the cytosolic side of the membrane, e.g. a multispan membrane protein like yeast Ste2 or yeast protein Fur3, yeast protein Alg5, or human or mouse G protein-coupled receptors like the beta2 adrenergic receptor. Also any type I integral membrane protein can be used, such as yeast Ost1, human or mouse amyloid precursor protein (APP), VEGF receptor isoforms 1, 2 or 3 (VEGFR1,VEGFR2, VEGFR3), epidermal growth factor receptor (EGFR), or yeast FET3. Preferably, a small integral membrane protein or a domain or a fragment of the proper orientation (C-terminus at the cytoplasmic side of the membrane) that spans the membrane only once is used, and most preferably it is the yeast Ost4 protein.

A further object of the present invention is to provide a method for the screening of libraries for identifying molecules, which may interact with transcriptionally active proteins, wherein the protein of interest must be artificially attached to the membrane.

Yet another object of the present invention is to provide a method which can be used to identify or design compounds or peptides or single chain antibodies that are capable of interfering with interactions between transcriptionally active proteins and other proteins, provided that the transcriptionally active protein is artificially attached to the membrane.

The system can also be used to test affinity reagents for protein purification.

The present invention also provides kits for carrying out each of the above-mentioned objects and a bait vector.

### Detailed description of the invention

These and other objects are achieved by the present invention, which provides a method and a kit for detecting interacting partners of transcriptionally active proteins and other proteins which are not suitable for being analysed by other methods such as e.g. by a classical yeast two-hybrid method. In order to make such proteins capable of being analysed for their interaction with partners by this method, two conditions must be met: 1) the bait protein must be targeted to the membrane and 2) the bait protein must be fused to the CbM-TA module containing the C-terminal domain of ubiquitin (CbM) followed e.g. by an artificial transcriptional activator (e.g. LexM-VP16). To guarantee the targeting of the bait protein to the membrane the bait protein must be fused at its N-terminus to a membrane anchor. This anchor can be an integral membrane protein or a membrane domain which has its C-terminus at the cytoplasmic side of the membrane. This anchor can be any eukaryotic or prokaryotic integral membrane protein or membrane-associated protein or a domain or a fragment of a membrane protein having its C-terminal end facing the cytosolic side of the membrane, e.g. a multispan membrane protein like yeast Ste2 or yeast protein Fur3, yeast protein Alg5, or human or mouse G protein-coupled receptors like the beta2 adrenergic receptor. Also any type I integral membrane proteins can be used, such as yeast Ost1, human or mouse amyloid precursor protein (APP), VEGF receptor isoforms 1, 2 or 3 (VEGFR1,VEGFR2, VEGFR3), epidermal growth factor receptor (EGFR), or yeast FET3. Preferably, a small integral membrane protein or a domain or a fragment of the proper orientation (C-terminus at the cytoplasmic side of the membrane) that spans the membrane only once is used, and most preferably it is the yeast Ost4 protein.

The term "NbM" as used herein refers to the N-terminal portion of yeast ubiquitin, which encompasses amino acids 1-37 of yeast ubiquitin. "NbM" contains either the amino acids 1-37 of wild type ubiquitin, or the amino acids 1-37 of mutated ubiquitin wherein e.g. the amino acid isoleucine at position 13 has been exchanged for any of the following amino acids: leucine, valine, alanine or glycine, or amino acids 1-37 of ubiquitin wherein the amino acid isoleucine at position 3 has been exchanged for any of the following amino acids: leucine, valine, alanine or glycine, or most preferably, amino acids 1-37 of ubiquitin wherein the amino acids isoleucine at position 3 and 13 have been exchanged for any of the following amino acids: leucine, valine, alanine or glycine.

The term "CbM" as used herein refers to the C-terminal portion of yeast ubiquitin, which encompasses amino acids 35-76 of yeast ubiquitin or amino acids 35-76 of mutated ubiquitin, wherein e.g. the amino acid lysine at position 48 has been changed to the amino acid glycine using standard site-directed mutatgenesis techniques (following, for instance, directions described in the STRATAGENE Quickchange Mutagenesis Kit, Stratagene, CA, USA). The exchange of lysine at position 48 for the inert glycine represents an important advantage over other variations of the split-ubiquitin technique (Dunnwald et al., 1999; Johnsson & Varshavsky, 1994; Laser et al., 2000; Stagljar et al., 1998; Wittke et al., 1999) because it prevents modification of CbM via the attachment of ubiquitin moieties. The poly-ubiquitination of unmodified CbM may interfere with the binding to NbM and may lead to the degradation of the bait polypeptide by enzymes of the ubiquitination pathway (Hershko & Ciechanover, 1992).

The term "bait" defines a protein or part thereof which is either transcriptionally active or difficult to be analyzed by other methods. In order to make such a protein capable of being analyzed for its interaction partners by this method, two conditions must be met: 1) the bait must be targeted to the membrane and 2) the bait must be fused to the CbM-TA module containing the C-terminal domain of ubiquitin (CbM) followed by an artificial transcriptional activator (e.g. LexM-VP16). To guarantee the targeting of the bait protein to the membrane the bait protein must be fused at its N-terminus to a membrane anchor. This anchor can be any eukaryotic or prokaryotic integral membrane protein or membrane-associated protein or a domain or a fragment of a membrane protein having its C-terminal end facing the cytosolic side of the membrane, e.g. a multispan membrane protein like yeast Ste2 or yeast protein Fur3, yeast protein Alg5, or human or mouse G protein-coupled receptors like the beta2 adrenergic receptor. Also any type I integral membrane protein can be used, such as yeast Ost1, human or mouse amyloid precursor protein (APP), VEGF receptor isoforms 1, 2 or 3 (VEGFR1,VEGFR2, VEGFR3), epidermal growth factor receptor (EGFR), or yeast FET3. Preferably, a small integral membrane protein or a domain or a fragment of the proper orientation (C-terminus at the cytoplasmic side of the membrane) that spans the membrane only once is used, and most preferably, the membrane anchor is the Ost4 yeast protein. The fusion of the bait protein to the membrane anchor and to the CdM-TA module is the "first hybrid protein".

The "first hybrid protein" defines a protein which comprises the membrane anchor at the N-terminus, then the bait protein at the C-terminus of the membrane anchor and then the CbM-TA module at the C terminus of the bait protein.

The "first chimeric gene" defines a gene comprising a DNA fragment encoding the first hybrid protein. Therefore it encodes a membrane anchor (e.g. Ost4) fused to the protein of interest (bait) fused to the CbM-TA module containing the C-terminal domain of ubiquitin (CbM) followed e.g. by an artificial transcriptional activator (e.g. LexM-VP16).

The term "bait vector" as used in this document refers to a nucleic acid construct which comprises regulatory sequences that are necessary for the transcription and translation of the encoded sequences by the host cell, and preferably regulatory sequences that are needed for the propagation of the nucleic acid construct in yeast and E. coli. The bait vector also comprises sequences encoding a membrane anchor and a CbM-TA module. Bait vector therefore allows cloning of the bait.

The term "prey" as used in this document defines a protein or part thereof that is to be tested for an interaction with the bait. It may be either a membrane bound or soluble protein.

The term "second hybrid protein" as used here defines a fusion between a prey polypeptide and one or more other polypeptides, one of which is a second protein sequence involved in intracellular protein degradation such as NbM. NbM may be either wild type NbM or a mutated version of NbM, wherein one or several of its amino acids have been replaced by other amino acids, as described in detail for "NbM" above.

The term "second chimeric gene" defines a gene encoding the second hybrid protein. It comprises a DNA sequence encoding a prey and a DNA sequence for the protein involved in intracellular protein degradation such as NbM.

The terms "prey vector" and "library vector" as used herein refer to a nucleic acid construct which comprises regulatory sequences that are necessary for the transcription and translation of the coding sequences by the host cell and a DNA sequence for the protein involved in intracellular protein degradation such as NbM, and preferably regulatory sequences that are needed for the propagation of the nucleic acid construct in yeast and E. coli. The prey vector allows cloning of the prey. If the prey vector is a cDNA or a genomic DNA or a synthetic DNA library, the vector is then called a "library vector".

The term "split-ubiquitin" as used herein refers to quasi-native yeast ubiquitin assembled from non-covalently linked NbM and CbM, which have been brought into close spatial proximity by the interaction of two unrelated polypetides that are fused to CbM and NbM, respectively. Split-ubiquitin is recognized by ubiquitin-specific proteases present in the cell, which attack the polypeptide chain C-terminal at the double glycine motif in CbM. The proteolytic cleavage leads to the breakage of the polypeptide chain after the double glycine motif.

The term "transactivator polypeptide" or "activator" as used in this document refers to any polypeptide that possesses the ability to activate the "reporter gene" of the host cell, e.g. by recruiting and activating the RNA polymerase II machinery of yeast. Preferably, the transactivator polypeptide is the acidic B42 domain and most preferably, it is the herpes simplex virus VP16 protein.

The term "LexM" as used in this document refers to the nucleic acid sequence encoding the bacterial repressor protein LexA or its translation product, wherein the sequence may either encode the wild type LexA sequence or a mutated LexA sequence wherein the amino acid arginine at position 157 has been replaced by a glycine or wherein the amino acid arginine at position 159 has been replaced by glutamate or glycine, or any combination of the two mutations.

The term "TA" or "artificial transcription factor" as used in this document refers to a hybrid protein consisting of (1) a polypeptide with the intrinsic ability to bind to a defined nucleic acid sequence, such as to the bacterial repressor protein LexM, the yeast Ga14 protein or the Drosophila melanogaster proteins Ubx and abd-A, and (2) any transactivator polypeptide, as defined above. Preferably TA is LexM-B42, Ga14-B42 or Ga14-VP16, and most preferably LexM-VP16.

The term "Ga14" as used in this document refers to the nucleic acid sequence encoding the DNA binding domain of the yeast protein Gal4.

The term "Gal1-74" as used in this document refers to the nucleic acid sequence encoding the yeast protein Gal4 fragment corresponding to amino acids 1-74 of its translation product.

The term "Gal1-93" as used in this document refers to the nucleic acid sequence encoding the yeast protein Gal4 fragment corresponding to amino acids 1-93 of its translation product.

The term "reporter strain" as used in this document refers to any host that contains at least one "reporter gene" such as a yeast strain that contains nucleic acid constructs, either integrated into its genome or as autonomously replicating elements, which produce a signal upon activation by the activator, e.g. by conferring the ability to grow on selective medium when activated, leading to cell death or cell cycle arrest when activated, or leading to production of enzymes such asp-galactosidase when activated. Preferably, the host cell comprises more than one reporter gene.

The term "reporter gene" as used in this document refers to a nucleic acid sequence comprising the following elements: (1) a binding site for an artificial transcription factor, (2) a minimal promoter sequence, which may be any sequence derived from a yeast promoter, but which preferably is any of GAL1 promoter, GAL2 promoter, CYC1 promoter, SP01 promoter, or HIS3 promoter, (3) a nucleic acid sequence encoding a polypeptide, which can be selected for or against in yeast or which has an enzymatic activity that can be measured using an appropriate assay, such as HIS3, ADE2, URA3, FAR1, or lacZ, and (4) a terminator sequence from a yeast gene, such as CYC1 or ADH1. The nucleic acid sequence may be integrated into the genome of a yeast reporter strain or it may supplied on an autonomously replicating plasmid.

The term "FAR1" refers to the nucleic acid sequence encoding the yeast FAR1 gene or to its gene product, wherein the amino acid serine at position 87 has been replaced by the amino acid proline in order to stabilize the protein and prevent its degradation by the proteasome machinery from yeast.

The term "5-FOA" as used in this document refers to the compound 5-fluoro-orotic acid, which may be converted into toxic metabolites by the action of the URA3 gene product, leading to cell death.

As used herein, "first protein or part thereof" refers to a bait protein. First protein or part thereof is a soluble protein that must be artificially bound to or integrated into the membrane by the fusion to a membrane anchor in order to be analysed by the method. In the context of the present invention, any soluble protein, both cytosolic and nuclear, or its part, subfragment or domain, can be used as the "first protein or part thereof". Even if the nuclear protein contains a strong nuclear localization signal, it will not be able to head on into the nucleus as it will be artificially anchored to the membrane. To guarantee the targeting of the bait protein to the membrane the bait protein must be fused at its N-terminus to a membrane anchor. This anchor can be any eukaryotic or prokaryotic integral membrane protein or membrane-associated protein or a domain or a fragment of a membrane protein having its C-terminal end facing the cytosolic side of the membrane, e.g. a multispan membrane protein like yeast Ste2 or yeast protein Fur3, yeast protein Alg5, or human or mouse G protein-coupled receptors like the beta2 adrenergic receptor. Also any type I integral membrane protein can be used, such as yeast Ost1, human or mouse amyloid precursor protein (APP), VEGF receptor isoforms 1, 2 or 3 (VEGFR1,VEGFR2, VEGFR3), epidermal growth factor receptor (EGFR),or yeast FET3. Preferably, a small integral membrane protein or a domain or a fragment of the proper orientation (C-terminus at the cytoplasmic side of the membrane) that spans the membrane only once is used, and most preferably it is the Ost4 yeast protein.

As used herein, the "second protein or part thereof" refers to a prey protein which is to be tested for its ability to interact with the "first membrane bound protein or part thereof" and which protein can be membrane bound or soluble. In the context of the present invention, a part of such protein is sufficient as long as it is capable of interacting with the "first membrane bound protein or part thereof", strong enough so that split-ubiquitin is formed.

"First protein sequence involved in intracellular protein degradation" and "second protein sequence involved in intracellular protein degradation" means parts of a protein which, when brought together in a host cell, e.g. by the interaction of the first protein to be tested and the second protein to be tested, reconstitutes a structure which is capable of activating an intracellular protein degradation machinery such as the ubiquitin depending proteases. For example, the first protein sequence may comprise the C-terminal part of ubiquitin (CbM) and the second protein sequence may comprise the N-terminal part of ubiquitin (NbM).

The term indicating that a protein has been "artificially attached to the membrane" or "artificially bound to the membrane" or "artificially anchored to the membrane" means that a protein was fused to a membrane anchor.

The term "membrane anchor" as used in this document refers to an integral membrane protein or a membrane domain that is used to target the bait protein to the membrane. This anchor can be any eukaryotic or prokaryotic integral membrane protein or membrane-associated protein or a domain or a fragment of a membrane protein having its C-terminal end facing the cytosolic side of the membrane, e.g. a multispan membrane protein like yeast Ste2 or yeast protein Fur3, yeast protein Alg5, or human or mouse G protein-coupled receptors like the beta2 adrenergic receptor. Also any type I integral membrane proteins can be used, such as yeast Ost1, human or mouse amyloid precursor protein (APP), VEGF receptor isoforms 1, 2 or 3 (VEGFR1,VEGFR2, VEGFR3), epidermal growth factor receptor (EGFR), or yeast FET3. Preferably, a small integral membrane protein or a domain or a fragment of the proper orientation (C-terminus at the cytoplasmic side of the membrane) that spans the membrane only once is used, and most preferably it is the Ost4 yeast protein.

The term "screening" as used here defines use of the method for simultaneous testing of a multiplicity of proteins to determine whether any interact with a bait protein. The second hybrid protein may be encoded in a library of plasmids that contain genomic DNA, cDNA or synthetically generated DNA sequences fused to the DNA sequence encoding the N-terminal domain of ubiquitin (NbM). Screening can be, for example, performed in such a way that a DNA fragment encoding the first hybrid protein is introduced into the host cell (yeast, bacterial or mammalian cells or any cell harbouring the reporter gene). For the second partner, a library of plasmids can be constructed which may include, for example, total human complementary DNA (cDNA) fused to the DNA sequence encoding the N-terminal domain of ubiquitin (NbM). This library is introduced into the host cell carrying bait protein. If any individual plasmid from the library encodes a protein that is capable of interacting with the bait protein, a positive signal will be obtained by activating the reporter gene. In addition, when an interaction between proteins occurs, the gene for the newly identified protein is available.

The present invention provides a method and a kit for detecting interacting partners of transcriptionally active proteins and other proteins which are not suitable for being analysed by other methods, e.g. by a classical yeast two-hybrid method. The invention makes use of reconstitution of split ubiquitin.

The present invention provides a method for detecting an interaction between a first protein or part thereof which is artificially bound to the membrane and a second protein or part thereof which is either membrane bound or soluble, the method comprising:
(a) providing a host cell containing at least one detectable gene (reporter gene) having a binding site for a transcriptional activator, such that the detectable gene expresses a detectable product, preferably a protein, when the detectable gene is transcriptionally activated;
(b) providing, as part of a bait vector, a first chimeric gene capable of being expressed in said host cell, the first chimeric gene coding inter alia for a first protein or part thereof which gene is attached to the DNA sequence of a membrane anchor and to the DNA sequence of the first module encoding inter alia a first protein sequence involved in intracellular protein degradation and a transcriptional activator, said first protein or part thereof to be tested whether it can interact with a second protein or part thereof;
(c) providing, as part of a prey vector, a second chimeric gene capable of being expressed in said host cell, the second chimeric gene coding inter alia for a second protein or part thereof which is either membrane bound or soluble and which gene is attached to the DNA sequence of a second module encoding inter alia a second protein sequence involved in intracellular protein in degradation;
(d) introducing the bait vector and the prey vector into the host cell such that an interaction between the expressed first and second protein and/or their parts can take place, which interaction leads to an interaction of the first protein sequence of the first module and the second protein sequence of the second module which interaction in turn leads to recognition by an intracellular protease and proteolytic separation of the transcriptional activator;
(e) determining whether the detectable gene of the host cell has been activated by the transcriptional activator.

The method is set up in yeast, preferably in Schizosaccharomyces pombe, most preferably in the budding yeast Saccharomyces cerevisiae, but can be set up as well in bacteria such as Escherichia coli or in mammalian cell systems or in any other type of cell that would allow the interaction between the bait and prey to take place and be detectable. The host cell therefore must contain a reporter gene having a binding site for a TA, such that the reporter gene expresses a detectable protein when the reporter gene is transcriptionally activated. Preferably TA is LexM-B42, Gal4-B42, or Ga14-VP16, and most preferably LexM-VP16. Also the host cell must allow expression of ubiquitin specific proteases (UBPs). Yeast and mammalian cells express UBPs. Other cell types that do not may have to be transformed with an appropriate construct encoding them.

The method makes use of chimeric genes, which express hybrid proteins. The first chimeric gene comprises a DNA fragment encoding a membrane anchor fused to the protein of interest (bait) fused to the CbM-TA module containing the C-terminal domain of ubiquitin (CbM) followed e.g. by an artificial transcriptional activator (e. g. LexM-VP16). Bait is a protein which is either transcriptionally active or difficult to be analysed by other methods. To guarantee the targeting of the first hybrid protein to the membrane the bait protein must be fused at its N-terminus to a membrane anchor. This anchor can be any eukaryotic or prokaryotic integral membrane protein or membrane-associated protein or a domain or a fragment of a membrane protein having its C-terminal end facing the cytosolic side of the membrane, e.g. a multispan membrane protein like yeast Ste2 or yeast protein Fur3, yeast protein Alg5, or human or mouse G protein-coupled receptors like the beta2 adrenergic receptor. Also any type I integral membrane proteins can be used, such as yeast Ost1, human or mouse amyloid precursor protein (APP), VEGF receptor isoforms 1, 2 or 3 (VEGFR1,VEGFR2, VEGFR3), epidermal growth factor receptor (EGFR), or yeast FET3. Preferably, a small integral membrane protein or a domain or a fragment of the proper orientation (C-terminus at the cytoplasmic side of the membrane) that spans the membrane only once is used, and most preferably the membrane anchor is the Ost4 yeast protein. Transcription of the first chimeric gene in a host cell ultimately leads to production of a first hybrid protein. The first hybrid protein therefore comprises the membrane anchor at the N-terminus, then the bait protein at the C-terminus of the membrane anchor and then the CbM-TA module at the C-terminus of the bait protein.

The second chimeric gene which is capable of being expressed in a host cell encodes the second hybrid protein. The second chimeric gene comprises a DNA sequence for a protein involved in intracellular protein degradation such as NbM and a DNA sequence that encodes a prey protein which is to be tested for interaction with the first protein or protein fragment. The second hybrid protein therefore comprises a fusion protein comprising a protein involved in intracellular protein degradation such as NbM fused to the test prey protein that can be a soluble or membrane-bound protein.

In order to test for interaction between the first hybrid protein and a second hybrid protein, the first chimeric gene and the second chimeric gene are introduced into a host cell, e.g. a yeast, bacterial or mammalian cell or any cell harbouring the reporter gene, most preferably a yeast reporter strain. The host cell is subjected to conditions under which the first protein and the second protein are expressed. If the two test proteins are able to interact, they reconstitute two separate ubiquitin domains which are now recognized by the UBPs. This leads to cleavage of the transcriptional activator and activation of the reporter gene(s). The cells are then tested for the expression of a reporter gene.

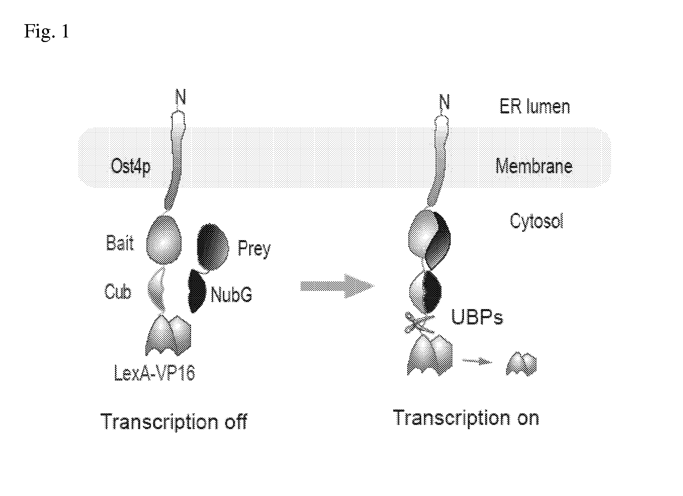

The basic strategy of the method is shown in Figure 1. A protein of interest (the bait) is N-inserted between the membrane protein Ost4 and the C-terminal half of ubiquitin (Cub which stands for CbM as defined above) followed by the artificial transcription factor LexM-VP16. A second protein (the prey) is fused to the mutated N-terminal half of ubiquitin (NubG which stands for NbM as defined above). If bait and prey interact, CbM and NbM complement to form split-ubiquitin, which is recognized and cleaved by UBPs. Cleavage leads to translocation of LexM-VP16 to the nucleus, where it activates LexM-responsive HIS3, ADE2 and lacZ reporter genes. Yeast cells harbouring an interacting protein pair are either selected for HIS3 or ADE2 prototrophy or by measuring beta-galactosidase activity.

One advantage of this method is that a multiplicity of proteins can be simultaneously tested to determine whether any interact with a bait protein, as the second hybrid protein may be encoded in a library of plasmids that contain genomic DNA, cDNA or synthetically generated DNA sequences fused to the DNA sequence encoding the N-terminal domain of ubiquitin (NbM). This so called screening can be for example performed in such a way that a DNA fragment encoding the first hybrid protein is introduced into the host cell (yeast, bacterial or mammalian cell or any cell harbouring the reporter gene). For the second partner, a library of plasmids can be constructed which may include, for example, total human complementary DNA (cDNA) fused to the DNA sequence encoding the N-terminal domain of ubiquitin (NbM).

This library is introduced into the host cell carrying bait protein. If any individual plasmid from the library encodes a protein that is capable of interacting with the bait protein, a positive signal will be obtained by activating the reporter gene. In addition, when an interaction between proteins occurs, the gene for the newly identified protein is available. Therefore the system can be of value in the identification of new genes. Many transcriptionally active proteins are involved in various diseases, especially cancer. Proteins that interact with such proteins may be discovered, and these proteins might turn out to be of therapeutic value.

The system can be used in the discovery or design of compounds or peptides or single chain antibodies that can interfere with the interaction between the first and the second hybrid protein. A compound or a peptide or a single chain antibody can be introduced into the host cell harbouring the first and the second hybrid protein. If this compound/peptide/single chain antibody can disturb interaction between the bait and prey, the activation of the reporter system will be negatively influenced.

The system can be used to test affinity reagents for protein purification. Peptides or protein domains can be identified that interact with the known transcriptionally active protein of interest and these may then be used in a purification protocol for the known protein.

The method of the present invention may be practiced using a kit for detecting binding between a first protein or part thereof which is artificially bound to the membrane and a second protein or part thereof which is either membrane bound or soluble, such kit comprising:
(a) a host cell containing at least one detectable gene (reporter gene) having a binding site for a transcriptional activator, such that the detectable gene expresses a detectable product, preferably a protein, when the detectable gene is transcriptionally activated;
(b) a first vector (bait vector) comprising a first site capable of receiving a first nucleic acid coding for a first protein or part thereof such that when the first nucleic acid is inserted it becomes attached to the DNA sequence of a membrane anchor and to the DNA sequence of the first module encoding inter alia a first protein sequence involved in intracellular protein degradation, the module further comprising a nucleic acid for a transcriptional activator;
(c) a second vector (prey vector) comprising a second site capable of receiving a second nucleic acid coding for a second protein or part thereof which is either membrane bound or soluble such that when the second nucleic acid is inserted it becomes attached to the DNA sequence of a second module encoding inter alia a second protein sequence involved in intracellular protein degradation.

The membrane anchor under b) can be any eukaryotic or prokaryotic integral membrane protein or membrane-associated protein or a domain or a fragment of a membrane protein having its C-terminal end facing the cytosolic side of the membrane, e.g. a multispan membrane protein like yeast Ste2 or yeast protein Fur3, yeast protein Alg5, or human or mouse G protein-coupled receptors like the beta2 adrenergic receptor. Also any type I integral membrane proteins can be used, such as yeast Ost1, human or mouse amyloid precursor protein (APP), VEGF receptor isoforms 1, 2 or 3 (VEGFR1,VEGFR2, VEGFR3), epidermal growth factor receptor (EGFR), or yeast FET3. Preferably, a small integral membrane protein or a domain or a fragment of the proper orientation (C-terminus at the cytoplasmic side of the membrane) is used, and most preferably it is the yeast Ost4 protein.

The kit may include vectors and a host cell. The vectors for the membrane based detection system include the bait vector and prey vector and optionally a plasmid library. A vector useful as a bait vector may comprise the following elements:
(1) a selection marker for propagation of the vector in E. coli;
(2) an origin of replication which allows propagation of the vector in E. coli;
(3) a further selection marker for propagation of the vector in yeast;
(4) an origin of replication which allows propagation of the vector in yeast; and
(5) an expression cassette comprising the following elements;
   (a) a promoter;
   (b) a nucleic acid sequence encoding a membrane anchor;
   (c) a site capable of receiving a nucleic acid encoding a protein;
   (d) a nucleic acid sequence encoding the C-terminal part of yeast ubiquitin (CbM);
   (e) a nucleic acid sequence encoding a DNA binding protein;
   (f) a nucleic acid sequence encoding a transcriptional activator; and
   (g) a terminator sequence.
      The membrane anchor under b) can be any eukaryotic or prokaryotic integral membrane protein or membrane-associated protein or a domain or a fragment of a membrane protein having its C-terminal end facing the cytosolic side of the membrane, e.g. a multispan membrane protein like yeast Ste2 or yeast protein Fur3, yeast protein Alg5, or human or mouse G protein-coupled receptors like the beta2 adrenergic receptor. Also any type I integral membrane proteins can be used, such as yeast Ost1, human or mouse amyloid precursor protein (APP), VEGF receptor isoforms 1, 2 or 3 (VEGFR1,VEGFR2, VEGFR3), epidermal growth factor receptor (EGFR), or yeast FET3. Preferably, a small integral membrane protein or a domain or a fragment of the proper orientation (C-terminus at the cytoplasmic side of the membrane) is used, and most preferably it is the yeast Ost4 protein.

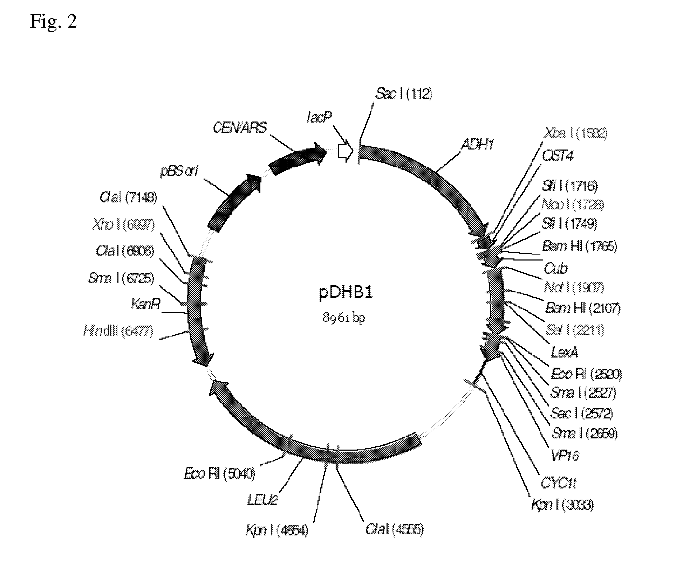

An exemplary bait vector pDHB 1 is shown in Figure 2. This specific vector embodiment comprises a yeast ADH1 promoter followed by a DNA sequence for the yeast Ost4 protein. A different yeast promoter could be used instead of the ADH1 promoter, e.g. a CYC1, CUP1, MET25, MET3 or a TEF promoter. Instead of the Ost4 sequence, a sequence for any eukaryotic or prokaryotic integral membrane protein or membrane-associated protein or a domain or a fragment of a membrane protein having its C-terminal end facing the cytosolic side of the membrane, e.g. a multispan membrane protein like yeast Ste2 or yeast protein Fur3, yeast protein Alg5, or human or mouse G protein-coupled receptors like the beta2 adrenergic receptor could be used. Also any type I integral membrane protein sequence could be used, such as the sequence for yeast Ost1, human or mouse amyloid precursor protein (APP), VEGF receptor isoforms 1, 2 or 3 (VEGFR1,VEGFR2, VEGFR3), epidermal growth factor receptor (EGFR), or yeast FET3. Also a sequence for a small integral membrane protein or a domain or a fragment of the proper orientation (C-terminus at the cytoplasmic side of the membrane) could be used.

The Ost4 sequence is followed by restriction sites for inserting a DNA sequence encoding a protein of interest in such a manner that the protein is expressed as a fusion to the CbM-LexM-VP16 portion. Instead of a CbM-LexM-VP16, any other artificial transcriptional activator could be used, such as CbM-LexM-B42 or CdM-Ga14-VP16, provided that the host cell comprises the appropriate biding sites for such transcriptional activator. In this particular embodiment the LexM sequence encodes a mutated sequence wherein the amino acid arginine at position 157 has been replaced by a glycine to reduce the nuclear localisation signal. This feature is often useful to reduce the number of so called "false positives" (host cells in which the activation of a reporter gene is not caused by a specific interaction between a bait and a prey), especially when a bait vector comprising a strong promoter such as ADH1 or TEF is used. For this purpose, also a LexA mutation wherein the amino acid arginine at position 159 has been replaced by glutamate or glycine, or any combination of the two mutations might be used as well. In another embodiment the wild type LexA sequence might be used, especially if a bait vector comprises a weak promoter such as CYC1 or CUP1. The vector also contains a terminator sequence that is necessary to terminate the transcription of a given protein, such as ADH1t or CYC1t. The vector in this embodiment includes a sequence that allows its replication in yeast. In this case, it is a CEN/ARS origin of replication. An alternative embodiment might be a vector with a 2micron origin of replication or an integrative vector that has to be stably integrated in the yeast genome. Also included on the vector is a first marker gene, the expression of which in the host cell permits selection of cells containing the first marker gene from cells that do not contain the first marker gene. This embodiment comprises a LEU2 marker, whereas in an alternative embodiment any other marker such as TRP1, HIS3, URA3 or KanMX might be used.

A prey vector (e. g. pX-HA-NbM), which allows the cloning of the prey protein (X), may be a transmembrane protein or soluble (cytoplasmic) protein. The test protein may be encoded in a library of plasmids that contain genomic DNA, cDNA or synthetically generated DNA sequences fused to the NbM domain. This vector also includes a promoter preferably selected from the group consisting of the ADH promoter, CYC1 promoter, MET3 promoter, MET25 promoter, CUP1 promoter and TEF promoter, and does include a transcription termination signal to direct transcription such as ADH1t or CYC1t. It also includes a DNA sequence that encodes the N-terminal domain of ubiquitin (NbM) and a unique restriction site to insert a DNA sequence encoding the second protein or protein fragment into the vector. Thus, this vector allows the cloning of the protein as an N-terminal fusion to the NbM domain. The vector further preferably includes a means for replicating itself in the host cell, i.e. yeast or bacteria. It also includes a second marker gene which is preferably the TRP1 marker, but could be any other yeast marker such as LEU2, HIS3, URA3 or KanMX, the expression of which in the host cell permits selection of cells containing the second marker gene from cells that do not contain the second marker gene.

A second prey vector (pNbM-HA-X) allows the cloning of the prey protein (X), a transmembrane protein or soluble (cytoplasmic) protein, as a C-terminal fusion to the NbM domain. The second prey vector also includes a promoter preferably selected from the group consisting of the ADH promoter, CYC1 promoter, MET3 promoter, MET25 promoter and TEF promoter, and a transcription termination signal to direct transcription such as ADH1t or CYC1t. It also includes a DNA sequence that encodes the N-terminal domain of ubiquitin (NbM) and a unique restriction site to insert a DNA sequence encoding the second protein or protein fragment into the vector. The second prey vector further preferably includes a means for replicating itself in the host cell and in bacteria. It also includes a second marker gene which is preferably the TRP1 marker, but could be any other yeast marker such as LEU2, HIS3, URA3 or KanMX, the expression of which in the host cell permits selection of cells containing the second marker gene from cells that do not contain the second marker gene.

The kit includes a host cell, preferably a yeast strain that contains a detectable gene having binding sites for the artificial transcription factor such as LexM-B42,r Ga14-B42 or Ga14-VP1 6, or most preferably LexM-VP16. The binding site is positioned so that the reporter gene expresses a reporter protein when two proteins (the first protein and the second protein) properly interact in this system. The host cell, by itself, is incapable of expressing a protein having a function of the first marker gene (LEU2), the second marker gene(TRP1), the CbM-TDA portion, or the NbM domain.

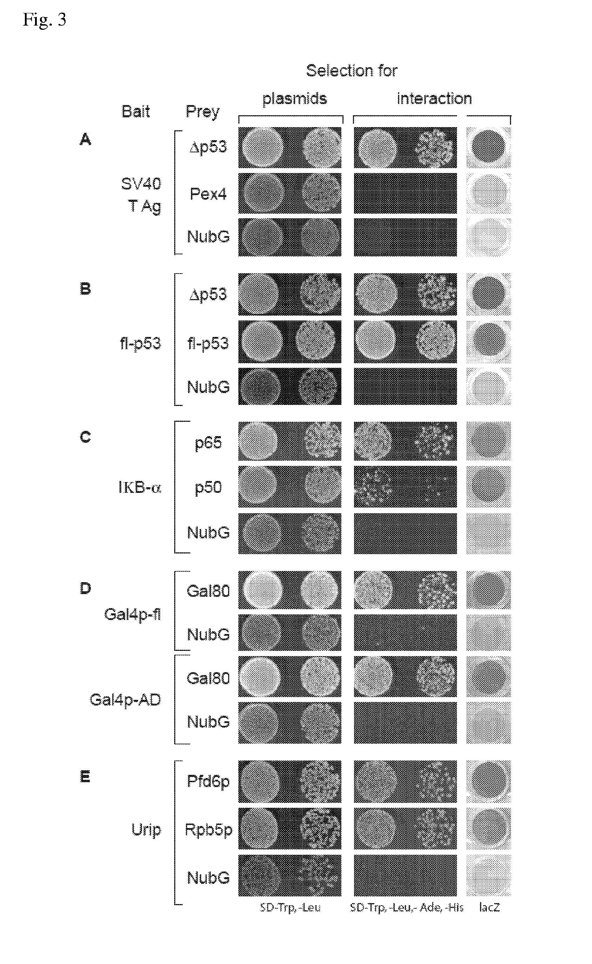

The method can be used for detection of interactions between transcriptionally active proteins or parts thereof and defined prey proteins, as is shown in Figure 3. Yeast co-expressing the indicated baits and preys are plated either on medium selecting for the presence of both bait and prey (left column) or on medium selecting for a protein interaction (right column). Beta-galactosidase activity of yeast transformants was measured by the pellet X-gal assay as described under Example 4. (A) Interaction between large T antigen and either delta-p53, Pex4p or NbM. (B) Interaction of full-length p53 with delta-p53, full-length p53 or NbM. (C) Interaction of IkappaB-alpha with p65, p50 or NbM. (D) Interaction of full-length Gal4p (Ga14p-f1) or the Gal4p activation domain (Ga14p-AD) with Gal80p or NbM. (E) Interaction of Urip with Pfd6p, Rpb5p or NbM.

The method can be used for screenings against cDNA libraries.
The following Table shows results of a screening for interactions with a large T antigen protein:

| Protein name | Number of identical clones found | Notes / subcellular localization |
|---|---|---|
| Emopamil binding protein 2 | 2 | Sterol isomerase, integral membrane protein, endoplasmic reticulum |
| Peroxiredoxin 4 | 1 | Thioredoxin-dependent peroxide reductase A0372, cytosolic |
| Unknown protein | 5 | Chromosome 6 ORF113, adjacent to importin-alpha6 gene |
| Importin alpha 2 subunit | 1 | Known large T Antigen Interactor, cytosolic and nuclear |
| Three prime repair exonuclease 1 | 1 | Exonuclease with a preference for double stranded DNA, nuclear |
| KIAA0033 | 1 | Transmembrane protein 41B |
| B-cell receptor associated protein 31 | 1 | Integral membrane protein, endoplasmic reticulum |

A human Jurkat T cDNA library in the prey vector pDSL-Nx (2micron vector in which cDNAs are expressed as fusions to the C-terminus of NbM, expression of cDNAs is driven by the CYC1 promoter) was transformed into the yeast reporter strain NMY32 harbouring pDHB1-large T antigen vector using the lithium acetate protocol as described under Example 2. Approximately 106 transformants were selected on SD-trp-leu-his-ade medium. Library plasmids were isolated from 45 positive clones and amplified in E .coli. Library clones were analyzed by restriction analysis for insert sizes, Western blotting and sequencing of the cDNA inserts. The identity of cDNA inserts was determined by performing BLAST searches (Altschul, S.F., W. Gish, W. Miller, E.W. Myers, and D.J. Lipman. 1990. J. Mol. Biol. 215:403-410) against GenBank.

### Examples

### Example 1: Bait and prey constructs

To construct the bait vector pDHB1 shown in Figure 2 the cDNA encoding the entire open reading frame of the yeast endoplasmatic reticulum OST4 was amplified by PCR from a genomic S. cerevisiae library using a forward primer with an Xba I site and a reverse primer with an Sfi I restriction site. The OST4 cDNA was inserted upstream of the multiple cloning site into the vector pCCW. The following bait proteins were cloned via Sfi I sites downstream of the OST4 sequence into pDHB1: N-terminally truncated Simian virus 40 large T antigen (amino acids 84-708), full-length human p53, full-length S. cerevisiae Urip (Systematic gene name YFL023w), full-length human IkappaB-alpha, full-length S. cerevisiae Gal4p, and the activation domain of Gal4p (amino acids 768-881). The following prey proteins were cloned downstream of the NbM into the prey vector pDSL-Nx (2micron vector in which prey proteins are expressed as fusions to the C-terminus of NbM and their expression is driven by the CYC1 promoter): truncated human p53 (amino acids 72-390), full-length human p53, full-length S. cerevisiae Ga180p, full-length human NF-kappaB subunit p65 and truncated human NF-kappaB subunit p50 (amino acids 1-354), full-length S. cerevisiae Pfd6p, and full-length S. cerevisiae Rpb5p. All constructs were verified by sequencing and the expression of all baits and preys was verified by Western blotting analysis.

### Example 2: Yeast transformation and spotting

The S. cerevisiae strain NMY32 [MATa trp1 leu2 his3 ade2 LYS2::LexA-HIS3 ade2::LexA-ADE2 URA3::LexA-lacZ] was co-transformed with bait and prey plasmids using the lithium acetate method (Gietz, R.D. and R.A. Woods. 2005. Methods Mol. Biol. 313:107-120). Transformants were selected for the presence of bait and prey plasmids during 3 days of growth at 30°C on SD-trp-leu medium (minimal medium containing 2% glucose (Sigma, Buchs, Switzerland), 0.67% yeast nitrogen base (BD Biosciences, Basel, Switzerland), complete amino acid mixture lacking leucine and tryptophan (Qbiogene, Basel, Switzerland), and 2% bacto agar (BD Biosciences). Several colonies were transferred to liquid SD-trp-leu medium and grown overnight to an absorbance (A₅₄₆) of 1.0. 45 microliters of different dilutions (1:100, 1:1000, and 1:10,000) were spotted onto SD-trp-leu and SD-trp-leu-his-ade medium (minimal medium as described above, but lacking tryptophan, leucine, histidine and adenine) and grown for 2 days at 30°C.

### Example 3: Beta-galactosidase assay

Beta-galactosidase activity was analyzed by the pellet X-gal assay as described previously (Mockli, N. and D. Auerbach. 2004. Biotechniques 36:872-876).

### Example 4: Detection of defined protein interactions.

The method can be used for detection of interactions between transcriptionally active proteins or parts thereof and defined prey proteins. Yeast co-expressing the indicated baits and preys are plated either on medium selecting for the presence of both bait and prey (Figure 3, left column) or on medium selecting for a protein interaction (right column). Beta-galactosidase activity of yeast transformants was measured by the pellet X-gal assay as described in Example 3.
To test whether the method is capable of detecting protein interactions between transcriptionally active proteins outside of the nucleus, namely on the side of the membrane facing the cytosol, a protein pair which is used as a positive control in most classical yeast two-hybrid systems was chosen, namely the Simian virus 40 large T antigen and the N-terminally truncated tumor suppressor protein p53 was selected (Schmieg, F.I. and D.T. Simmons. 1984. J. Virol. 52:350-355). To reconstruct the interaction between p53 and large T antigen, large T antigen was fused to Ost4p and CbM-LexM-VP16 as a bait and deltap53 was fused to NbM for use as prey. Co-expression of the large T antigen bait and the deltap53 prey yielded robust growth under selection and strong blue coloration in a beta-galactosidase assay (Fig. 3A). The interaction was specific since the large T antigen bait interacted neither with the unrelated prey Pex4p, nor with NbM expressed from the empty prey vector (Fig. 3A). To demonstrate that transcriptionally active full-length p53 can also be used in the method, the dimerization of p53 was assayed (Levine, A.J. 1997. Cell 88:323-331). Due to its intrinsically self-activating transactivation domain at the N-terminus, full-length p53 cannot be used as a bait in a classical yeast two-hybrid assay (Chen, S.S., P.C. Chang, Y.W. Cheng, F.M. Tang, and Y.S. Lin. 2002. Embo J. 21:4491-4499). Instead, the N-terminally truncated variant (deltap53) is normally used. Here a full-length p53 bait was constructed and tested for its interaction with a full-length p53 prey and a deltap53 prey. The full-length p53 bait interacted strongly with both deltap53 prey and full-length p53 prey, but not with NbM expressed from the empty prey vector (Fig. 3B). These results suggest that the method can detect interactions involving nuclear proteins and more importantly, that full-length, transcriptionally active proteins can be used in this assay.
To investigate whether the system can also be applied to other transcription factors, interactions involving proteins of the NF-kappaB complex were investigated. NF-kappaB subunits p65 and p50 and their inhibitor IkappaB-alpha (Jacobs, M.D. and S.C. Harrison. 1998. Cell 95:749-758) were selected since the interaction is well characterized and p65 and p50 are notoriously difficult to handle in yeast two-hybrid assays. Upon co-expression, the specific association of IkappaB-alpha bait with either p65 or p50 prey was clearly detectable (Fig. 3C). IkappaB-alpha has a higher affinity for p65 than for p50 and this difference in affinity was clearly detectable through enhanced growth of IkappaB-alpha /p65 transformants when compared to IkappaB-alpha /p50 transformants. Also a classical yeast transcription factor can be used with this system. To prove this, the interaction between Gal4p and its repressor Gal80p was chosen. Gal4p cannot be used in a conventional yeast two-hybrid system since it strongly stimulates transcription in the absence of a protein interaction when used as bait (Lohr, D., P. Venkov, and J. Zlatanova. 1995. Faseb J. 9:777-787). In fact, the classical yeast two-hybrid system is often based on the use of the DNA-binding and activation domains of Gal4p to detect a protein interaction (Fields, S. and O. Song. 1989. Nature 340:245-246.). Both full-length Gal4p and its activation domain alone were tested for interaction with Ga180p. Full-length Gal4p and the Gal4p activation domain alone interacted specifically with the Gal80p prey, but not with NbM alone (Fig. 3D).
A surprisingly high number of cytosolic proteins also autonomously activate transcription when used as baits in the classical yeast two-hybrid system, most likely due to the presence of clusters of acidic residues in their primary sequence. To test whether this system can be used to investigate interactions involving such proteins, the yeast prefoldin-like protein Urip (Gstaiger, M., B. Luke, D. Hess, E.J. Oakeley, C. Wirbelauer, M. Blondel, M. Vigneron, M. Peter, and W. Krek. 2003. Science 302:1208-1212) was chosen as a bait. First, Urip was tested in a conventional yeast two-hybrid system and it proved unsuitable for it, presumably due its strongly acidic C-terminal region. As shown in Fig. 3E, full-length Urip bait interacted with two of its known binding partners, Pfd6p and Rpb5p, when tested in the method of this patent application. Urip, Pfd6p and Rpb5p are all found in the cytosol (Huh, W.K., J.V. Falvo, L.C. Gerke, A.S. Carroll, R.W. Howson, J.S. Weissman, and E.K. O'Shea. 2003. Nature 425:686-691; Delgermaa, L., N. Hayashi, D. Dorjsuren, T. Nomura, T.T. Thuyle, and S. Murakami. 2004. Mol. Cell. Biol. 24:8556-8566). Thus, this system can also be used to detect interactions between cytosolic proteins that are unsuitable for use with the conventional yeast two-hybrid system (right column). Detection of the protein interaction is achieved by measuring the output of the activated reporter genes (either by growth on selective medium or by blue coloration in a beta-galactosidase assay). Beta-galactosidase activity of yeast transformants was measured by the pellet X-gal assay as described under Example 3.

### Example 5: Screening procedure

The most widely used application of genetic screening systems in yeast is the identification of novel interaction partners through cDNA library screening. To investigate whether the system of this invention is also suitable for this purpose, the large T antigen was used as a bait to screen a NbM-fused Jurkat cell cDNA library. A human Jurkat T cDNA library in the prey vector pDSL-Nx (2micron vector in which cDNAs are expressed as fusions to the C-terminus of NbM, expression of cDNAs is driven by the CYC1 promoter) was transformed into the yeast reporter strain NMY32 harbouring pDHB1-large T antigen using the lithium acetate protocol as described under Example 2. One million clones were screened. Approximately 106 transformants were selected on SD-trp-leu-his-ade medium. Library plasmids were isolated from 45 positive clones and amplified in E. coli. Library clones were analyzed by restriction analysis for insert sizes, Western blotting and sequencing of the cDNA inserts. The identity of cDNA inserts was determined by performing BLAST searches (Altschul, S.F., W. Gish, W. Miller, E.W. Myers, and D.J. Lipman. 1990. J. Mol. Biol. 215:403-410) against GenBank. The results of the screening are presented in the Table.

Seven putative interactors were identified. Six clones encoded previously unknown interactors (Table), whereas one clone encoded a known binding partner of large T antigen, importin alpha-2. Importin alpha-2 is involved in nuclear import of the viral protein (Catimel, B., T. Teh, M.R. Fontes, I.G. Jennings, D.A. Jans, G.J. Howlett, E.C. Nice, and B. Kobe. 2001. J. Biol. Chem. 276:34189-34198). Another interactor, peroxiredoxin 4, has several previously published functional links to large T antigen. For example, members of the peroxiredoxin family are involved in the inhibition of simian virus and HIV virus replication in CD8(+) T-cells (Geiben-Lynn, R., M. Kursar, N.V. Brown, M.M. Addo, H. Shau, J. Lieberman, A.D. Luster, and B.D. Walker. 2003. J. Biol. Chem. 278:1569-1574). The isolation of importin alpha-2 and several other putative interactors demonstrates that this method can be used to identify interactors of a protein of interest by cDNA library screening.

### Example 6: Bait vectors with signal sequences for N-terminal fatty acid modification such as myristoylation and palmytoylation cannot be used in the system.

Many proteins, such as G protein alpha subunits, are modified at or near their N termini by covalent attachment of the fatty acids myristate and/or palmitate. Fatty acid modification leads to targeting and anchoring of those proteins at the inner leaflet of the plasma membrane (Wedegaertner et al. 1995. J. Biol. Chem. 270: 503-506). Myristoylation is the result of co-translational addition of the saturated 14-carbon fatty acid myristate to a glycine residue at the extreme N terminus after removal of the initiating methionine. In contrast, palmytoylation is the addition of 16-carbon, saturated fatty acid attached through a labile, reversible thioester linkage to a cysteine near the N terminus. Myristoylation and palmytoylation can occur separately on a target protein or can be combined in the same protein. For G proteins, as well as other lipid-modified proteins, attached lipids appear to direct the interaction with membrane lipids, often leading to attachment of the modified protein to the lipid bilayer. Fatty acid modification sequences mediating myristoylation or palmytoylation can be artificially attached to proteins normally lacking those sequences, either by copying naturally occurring fatty acid modification sequences (such as those found in many G protein subunits) or by creating new fatty acid modification sequences from naturally occurring ones (Angermayr et al. 2000. FEBS Lett. 8;481(1):8-12). For example, the artificial myristoylation/palmytoylation signal MGCTVSS has been shown to efficiently target proteins to the membrane that are not normally localized there (Angermayr et al. 2000. FEBS Lett. 8;481(1):8-12).
To investigate whether fatty acid modification would be sufficient to target a heterologous protein to the membrane in yeast, which is expressed as a fusion to the CbM- LexM-VP16, and to avoid translocation of said protein to the nucleus and consequent activation of the LexM operator-driven reporter genes integrated into the yeast genome, the following experiments were carried out: A bait vector pCAS-MP was created by cloning a short DNA sequence encoding the amino acid sequence MGCTLSAEDKPGGPRSATSLQGC upstream of the cDNA sequence encoding CbM-LexM-VP16 into the basic bait vector pCAS-flv. This sequence is an artificial fatty acid modification signal, composed of a myristoylation and a palmytoylation signal, which has been shown to allow effective targeting of heterologous proteins to the membrane. To create two bait constructs for testing, the cDNA sequence encoding full-length human NF-kappaB subunit p65 was cloned into pCAS-MP to yield the bait vector 1. The full-length sequence of human plakoglobin was cloned into pCAS-MP to yield the bait construct 2. p65 is known to carry several strong nuclear localization signals, resulting in strong nuclear translocation of exogenously expressed p65, whereas plakoglobin is known to carry a weaker nuclear localization signal, resulting in intermediate nuclear translocation of exogenously expressed plakoglobin. Thus, in the absence of a fatty acid modification, both a p65 protein and a plakoglobin protein would be translocated to the nucleus, resulting in activation of the reporter genes and the inability to screen these proteins in the system. The expectation was that the fatty acid modification should efficiently target both the p65 hybrid protein and the plakoglobin hybrid protein to the membrane, thereby avoiding activation of the reporter gene by translocation of the proteins to the nucleus. To test this, p65 hybrid was co-expressed with (1) an empty library vector expressing unfused NbM and (2) an empty vector control. In both cases, strong activation of the reporter genes was observed, indicating that, regardless of the fatty acid modification, the p65 hybrid protein was translocating to the nucleus. The identical experiment was carried out using the plakoglobin hybrid protein. Again, activation of the reporter genes was observed when plakoglobin hybrid protein was coexpressed with either unfused NbM or empty vector. These results strongly suggest that even a double fatty acid modification (myristoylation and palmytoylation) is not sufficient to avoid nuclear translocation of a protein carrying a strong nuclear localization signal. The reason for the inefficient targeting to the membrane is probably the fact that the fatty acid modification is carried out post-translationally, allowing significant amounts of unmodified hybrid protein to escape to the nucleus and (2) the fact that fatty acid modifications are reversible, leading to an equilibrium of membrane-bound and non-bound hybrid proteins in the cell. Contrary to this, fusing a protein with a strong nuclear localization signal such as p53, IkappaB-alpha, Gal4 and large T-antigen (as shown in the previous examples) to the Ost4 resulted in hybrid proteins which could be used for detecting their interacting partners in this system. It is obvious that the N-terminal membrane anchor in the form of Ost4 or any other membrane protein or domain that has its C-terminus on the cytoplasmic side guarantees a stable expression and successful targeting of the proteins to the membrane. This might be due to the fact that the N-terminal membrane anchor is not a reversible modification and that it is, being at the N-terminus, translated first leading the whole hybrid protein in direction of the membrane so that the hybrid protein is already inserted into the membrane during the translation.

### References

Altschul, S.F., W. Gish, W. Miller, E.W. Myers, and D.J. Lipman. 1990. J. Mol. Biol. 215:403-410.
Angermayr et al. 2000. FEBS Lett. 481(1):8-12.
Catimel, B., T. Teh, M.R. Fontes, I.G. Jennings, D.A. Jans, G.J. Howlett, E.C. Nice, and B. Kobe. 2001. J. Biol. Chem. 276:34189-34198.
Chen, S.S., P.C. Chang, Y.W. Cheng, F.M. Tang, and Y.S. Lin. 2002. Embo J. 21:4491-4499. Delgermaa, L., N. Hayashi, D. Dorjsuren, T. Nomura, T.T. Thuy le, and S. Murakami. 2004. Mol. Cell. Biol. 24:8556-8566.
Dunnwald, M., Varshavsky, A., & Johnsson, N. (1999) Mol Biol Cell 10, 329-44.
Fashena, S.J., I. Serebriiskii, and E.A. Golemis. 2000. Gene 250:1-14.
Fields, S. and Song, O.K., 1989, Nature 340, 245-248.
Gietz, R.D. and R.A. Woods. 2005. Methods Mol. Biol. 313:107-120.
Geiben-Lynn, R., M. Kursar, N.V. Brown, M.M. Addo, H. Shau, J. Lieberman, A.D. Luster, and B.D. Walker. 2003. J. Biol. Chem. 278:1569-1574).
Gstaiger, M., B. Luke, D. Hess, E.J. Oakeley, C. Wirbelauer, M. Blondel, M. Vigneron,M. Peter, and W. Krek. 2003. Science 302:1208-1212.
Hershko, A., & Ciechanover, A. (1992) Annu Rev Biochem 61, 761-807.
Huh, W.K., J.V. Falvo, L.C. Gerke, A.S. Carroll, R.W. Howson, J.S. Weissman, and E.K. O'Shea. 2003. Nature 425:686-691.
Jacobs, M.D. and S.C. Harrison. 1998. Cell 95:749-758.
Johnsson, N. and Varshavsky, A. 1994. Proc. Natl. Acad. Sci. USA 91, 10340-10344.
Laser, H., Bongards, C., Schuller, J., Heck, S., Johnsson, N., & Lehming, N. (2000) Proc Natl Acad Sci USA 97, 13732-7.
Levine, A.J. 1997. Cell 88:323-331.
Lohr, D., P. Venkov, and J. Zlatanova. 1995. Faseb J. 9:777-787.
Mockli, N. and D. Auerbach. 2004. Biotechniques 36:872-876.
Schmieg, F.I. and D.T. Simmons. 1984. J. Virol. 52:350-355.
Solski et al., Methods in Enzymol. 250: 435-54, 1995.
Stagljar et al., Proc. Natl. Acad. Sci., USA 95, p. 5187-5192,1998)
Wedegaertner et al. (1995) J. Biol. Chem., 270: 503-506.
Wittke, S., Lewke, N., Muller, S., & Johnsson, N. (1999) Mol Biol Cell 10, 2519-30.

## Claims

1. A method for detecting an interaction between a first protein or part thereof which is artificially bound to the membrane and a second protein or part thereof which is either membrane bound or soluble, the method comprising:
(a) providing a host cell containing at least one detectable gene (reporter gene) having a binding site for a transcriptional activator, such that the detectable gene expresses a detectable product, preferably a protein, when the detectable gene is transcriptionally activated;
(b) providing, as part of a bait vector, a first chimeric gene capable of being expressed in said host cell, the first chimeric gene coding inter alia for a first protein or part thereof which gene is attached to the DNA sequence of a membrane anchor and to the DNA sequence of the first module encoding inter alia a first protein sequence involved in intracellular protein degradation and a transcriptional activator, said first protein or part thereof to be tested whether it can interact with a second protein or part thereof;
(c) providing, as part of a prey vector, a second chimeric gene capable of being expressed in said host cell, the second chimeric gene coding inter alia for a second protein or part thereof which is either membrane bound or soluble and which gene is attached to the DNA sequence of a second module encoding inter alia a second protein sequence involved in intracellular protein in degradation;
(d) introducing the bait vector and the prey vector into the host cell such that an interaction between the expressed first and second protein and/or their parts can take place, which interaction leads to an interaction of the first protein sequence of the first module and the second protein sequence of the second module which interaction in turn leads to recognition by an intracellular protease and proteolytic separation of the transcriptional activator;
(e) determining whether the detectable gene of the host cell has been activated by the transcriptional activator.

2. The method according to claim 1, wherein the host cell is a yeast, a bacterial or a mammalian cell.

3. The method according to claim 2, wherein the host cell is a Saccharomyces pombe cell or, more preferably, a cell of the budding yeast Saccharomyces cerevisiae.

4. The method according to any of claims 1 to 3, wherein the detectable gene is activated by the artificial transcriptional activator LexM-V 16.

5. The method according to any of claims 1 to 4, wherein the first protein sequence comprises a C-terminal portion of ubiquitin CbM and the second protein sequence comprises an N-terminal portion of ubiquitin NbM.

6. The method according to any of claims 1 to 5, wherein the DNA sequence coding for the first protein codes for transcriptionally active proteins or parts thereof.

7. The method according to any one of the preceding claims wherein the second membrane protein or soluble protein is encoded by a library of plasmids.

8. The method according to any one of the preceding claims wherein the bait and the prey vector are suitable for being maintained episomally.

9. The method according to any one of the preceding claims wherein the membrane anchor is any membrane protein or a membrane domain having its C-terminus in the cytoplasm.

10. The method according to any one of the preceding claims wherein the membrane anchor is a type I membrane protein, preferably Ost4.

11. A kit for detecting binding between a first protein or part thereof which is artificially bound to the membrane and a second protein or part thereof which is either membrane bound or soluble comprising:
(a) a host cell containing at least one detectable gene (reporter gene) having a binding site for a transcriptional activator, such that the detectable gene expresses a detectable product, preferably a protein, when the detectable gene is transcriptionally activated;
(b) a first vector (bait vector) comprising a first site capable of receiving a first nucleic acid coding for a first protein or part thereof such that when the first nucleic acid is inserted it becomes attached to the DNA sequence of a membrane anchor and to the DNA sequence of the first module encoding inter alia a first protein sequence involved in intracellular protein degradation, the module further comprising a nucleic acid for a transcriptional activator;
(c) a second vector (prey vector) comprising a second site capable of receiving a second nucleic acid coding for a second protein or part thereof which is either membrane bound or soluble such that when the second nucleic acid is inserted it becomes attached to the DNA sequence of a second module encoding inter alia a second protein sequence involved in intracellular protein degradation.

12. The kit according to claim 11, wherein the host cell is a yeast, bacterial or mammalian cell.

13. The kit according to claim 11, wherein the host cell is a yeast cell, preferably of Saccharomyces pombe and most preferably of the budding yeast Saccharomyces cerevisiae.

14. The kit according to any of claims 11 to 13, wherein the detectable gene can be activated by a natural or artificial activator, preferably an activator comprising a short tagging module.

15. The kit according to any of claims 11 to 13, wherein the detectable gene can be activated by the artificial transcriptional activator LexM-V 16.

16. The kit according to any of claims 11 to 15, wherein the first protein sequence contains the C-terminal part of ubiquitin CbM and the second protein sequence contains the N-terminal part of ubiquitin NbM.

17. The kit according to any of claims 11 to 16, wherein the DNA sequence coding for the second protein is contained in a library of plasmids.

18. The kit according to any of claims 11 to 17, wherein the membrane anchor is any membrane protein or a membrane domain having its C-terminus in the cytoplasm.

19. The kit according to any of claims 11 to 18, wherein the membrane anchor is a type I membrane protein, preferably Ost4.

20. A vector useful as a bait vector in a method according to any of claims 1 to 10 or a kit according to any of claims 11 to 19 comprising the following elements:
(1) a selection marker for propagation of the vector in E. coli;
(2) an origin of replication which allows propagation of the vector in E. coli;
(3) a further selection marker for propagation of the vector in yeast;
(4) an origin of replication which allows propagation of the vector in yeast; and
(5) an expression cassette comprising the following elements;
(a) a promoter;
(b) a nucleic acid sequence encoding a membrane anchor;
(c) a site capable of receiving a nucleic acid encoding a protein;
(d) a nucleic acid sequence encoding the C-terminal part of yeast ubiquitin (CbM);
(e) a nucleic acid sequence encoding a DNA binding protein;
(f) a nucleic acid sequence encoding a transcriptional activator; and
(g) a terminator sequence.

21. The vector according to claim 20, wherein the nucleic acid sequence encoding a membrane anchor according to element (5b) is a nucleic acid sequence encoding any membrane protein or a membrane domain having its C-terminus in the cytoplasm.

22. The vector according to claim 20, wherein the nucleic acid sequence encoding a membrane anchor according to element (5b) is a nucleic acid sequence encoding a type I membrane protein, preferably Ost4.
